(19) Europäisches Patentamt
European Patent Office
Office européen des brevets

(11) Veröffentlichungsnummer: **0 094 343**
**A1**

(12) # EUROPÄISCHE PATENTANMELDUNG

(21) Anmeldenummer: **83810168.1**

(22) Anmeldetag: **21.04.83**

(51) Int. Cl.³: **C 07 D 211/46,** C 07 D 211/58, C 07 D 211/94, C 07 D 471/10, C 07 D 491/113, C 07 D 498/10, C 08 K 5/34, C 08 K 5/35
//
C09D7/12 ,(C07D471/10, 235/00, 221/00),(C07D491/113, 319/00, 221/00),(C07D491/113, 317/00, 221/00),(C07D498/10, 263/00, 221/00)

(30) Priorität: **27.04.82 CH 2567/82**

(43) Veröffentlichungstag der Anmeldung: **16.11.83**
**Patentblatt 83/46**

(84) Benannte Vertragsstaaten: **CH DE FR GB IT LI**

(71) Anmelder: **CIBA-GEIGY AG, Patentabteilung Postfach, CH-4002 Basel (CH)**

(72) Erfinder: **Karrer, Friedrich, Dr., Rebbergstrasse 5, CH-4800 Zofingen (CH)**

(54) **Isocyanatgruppenhaltige Polyalkylpiperidinderivate.**

(57) Verbindungen der Formeln I, II, Ia und IIa

$$A-\overset{O}{\overset{\|}{C}}-NH-B-(NCO)_m \qquad I,$$

$$(OCN)_m-B-NH-\overset{O}{\overset{\|}{C}}-D-\overset{O}{\overset{\|}{C}}-NH-B-(NCO)_m \qquad II,$$

$$A'-\overset{O}{\overset{\|}{C}}-NH-B-(NCO)_m \qquad Ia,$$

$$(OCN)_mB-NH-\overset{O}{\overset{\|}{C}}-D'-\overset{O}{\overset{\|}{C}}-NH-B-(NCO)_m \qquad IIa,$$

worin m 1-3 ist, A und A' einwertige Polyalkylpiperidinreste, D und D' zweiwertige Polyalkylpiperidinreste und B den (m + 1)-wertigen Rest eines Polyisocyanates bedeuten, können aus den entsprechenden Polyalkylpiperidinen durch Umsetzung mit einem Überschuss eines Polyisocyanates hergestellt werden. Die Verbindungen sind Lichtschutzmittel und können über ihre Isocyanatgruppen an das zu schützende Substrat chemisch gebunden werden.

EP 0 094 343 A1

0094343



CIBA-GEIGY AG
Basel (Schweiz)

3-13894/S/=

Isocyanatgruppenhaltige Polyalkylpiperidinderivate

Die Erfindung betrifft neue isocyanatgruppenhaltige Polyalkylpiperidinderivate, die Lichtschutzmittel für organische Materialien darstellen und die durch ihre Isocyanatgruppen mit den zu schützenden Materialien chemisch verknüpft werden können.

Es ist allgemein bekannt, dass Piperidinderivate, die in 2- und 6-Stellung tetraalkyliert sind, wirksame Lichtschutzmittel für lichtempfindliche organische Materialien, insbesondere für organische Polymere, sind. Es ist weiterhin bekannt, dass die Flüchtigkeit und Wasserlöslichkeit einfacher Polyalkylpiperidine zu hoch ist, um einen lange andauernden Lichtschutz der zu schützenden Materialien zu gewährleisten, insbesondere, wenn die Materialien erhöhten Temperaturen ausgesetzt werden. Man hat deshalb versucht, durch entsprechende Substituenten in 4-Stellung des Piperidinringes das Molekulargewicht zu erhöhen und die Flüchtigkeit und Wasserlöslichkeit zu erniedrigen. Ein weiterer Schritt in dieser Richtung war die Schaffung polymerer oder oligomerer Polyalkylpiperidinderivate wie sie beispielsweise in der DE-A 27 19 131 oder EP-A 496 beschrieben sind. Es wurden auch schon reaktive Derivate von Polyalkylpiperidinen vorgeschlagen, die mit funktionellen Gruppen der zu schützenden Materialien reagieren können und so einen dauerhaften Lichtschutz gewährleisten. Beispiele hierfür sind die in der DE-A 26 42 446 oder die in der US-PS 4,234,728 beschriebenen N-Methylolderivate. In der DE-A 29 50 067 wurden auch bereits isocyanatgruppenhaltige Polyalkylpiperidinderivate der Formel

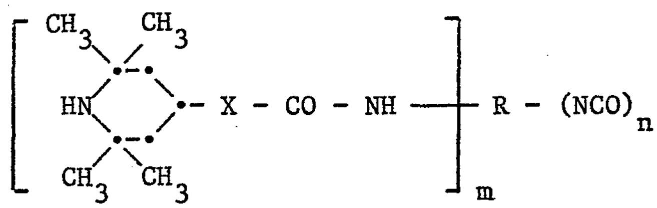

beschrieben, worin X -O-, -S- oder -NH-, R der Rest eines Polyisocyanates und m und n Werte grösser als 1 und kleiner als 5 sind. Solche Verbindungen können mittels ihrer Isocyanatgruppen mit bestimmten Substraten chemisch verknüpft werden und diese damit langdauernd stabilisieren. Ausserdem sind diese Verbindungen aufgrund ihrer NH-Gruppe in 1-Stellung des Piperidinringes als nicht-auswandernde Katalysatoren bei der Herstellung von Polyurethanschaumstoffen und als Vernetzer für Epoxidharze geeignet.

Bei der Herstellung dieser Verbindungen zeigt sich jedoch, dass die NH-Gruppe in 1-Stellung trotz der sterischen Hinderung in der Lage ist, mit den Isocyanatgruppen des eigenen Moleküles zu reagieren, wodurch es zur Bildung von oligomeren Harnstoffen kommt. Beispielsweise bilden sich bei der Umsetzung von 4-Amino-2,2,6,6-tetramethylpiperidin und Diisocyanaten auch bei Vorliegen eines Uberschusses des Diisocyanates Oligomere vom Typ

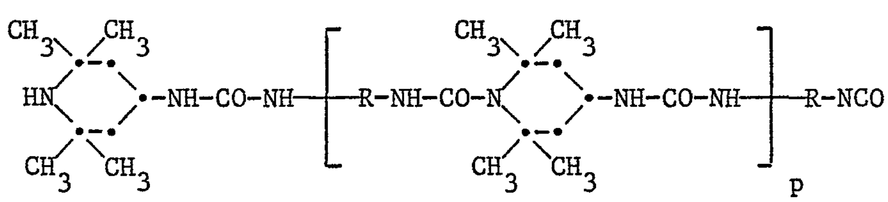 ,

wobei p je nach Verfahrensbedingungen Werte von etwa 2-4 annimmt. Dies geht aus den Beispielen der DE-A 26 42 446 auf Seite 12-14 hervor, wo beschrieben wird, dass die Produkte einen Isocyanatgehalt aufweisen, der etwa 20-30% des für das 1:1-Addukt berechneten Wertes ausmacht. Wie ebenfalls in diesen Beispielen beschrieben wird, sind die Produkte in organischen Lösungsmitteln schwer löslich. Sie sind damit nicht zur Stabilisierung von gelösten Materialien wie z.B. von

Lacken geeignet. Es besteht dabei auch die Gefahr, dass ein solcher Stabilisator während der Lagerung oder nach dem Zusatz zum zu stabilisierenden Material durch weitere Polyaddition sein Molekulargewicht erhöht und damit seine Löslichkeit und Verträglichkeit im Substrat weiter absinkt. Ein weiterer Nachteil der beschriebenen Isocyanatopiperidine ist, dass es sehr schwierig ist, ein solches Produkt in reproduzierbarer Qualität, insbesondere mit reproduzierbarem Molekulargewicht, herzustellen.

Es wurde gefunden, dass diese Schwierigkeiten nicht auftreten, wenn man von Aminogruppen- oder Hydroxylgruppen-haltigen Polyalkylpiperidinen ausgeht, die in der 1-Stellung N-substituiert sind. Dadurch wird verhindert, dass das Polyisocyanat während der Herstellung am Stickstoff der 1-Stellung reagieren kann und die so erhaltenen Produkte sind bei Lagerung und Einarbeitung in das zu schützende Material molekulargewichtsstabil. Trotzdem behalten sie die Fähigkeit, auf Grund ihrer freien Isocyanatgruppen mit funktionellen Gruppen des Substrates zu reagieren und dadurch ein Auswandern aus dem Substrat zu verhindern.

Gegenstand der Erfindung sind daher Verbindungen der Formel I oder II

$$A - \overset{\overset{O}{\|}}{C} - NH - B - (NCO)_m \qquad I$$

$$(OCN)_m - B - NH - \overset{\overset{O}{\|}}{C} - D - \overset{\overset{O}{\|}}{C} - NH - B - (NCO)_m \qquad II \quad ,$$

worin m 1 bis 3 ist,

A ein einwertiger Piperidinrest der Formeln III bis X ist,

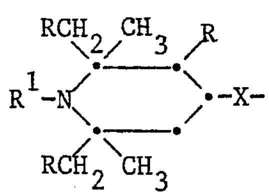

III

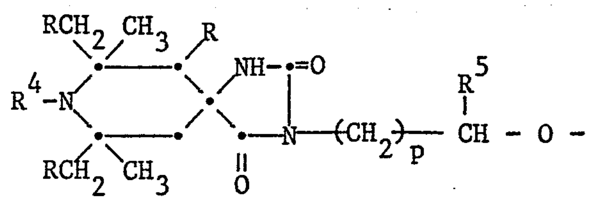

IV

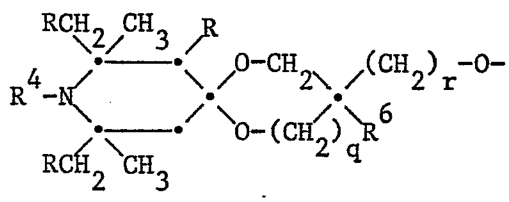

V

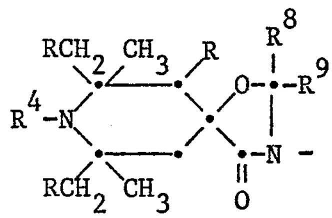

VI

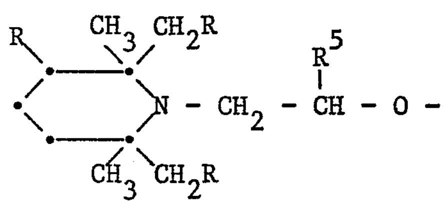

VII

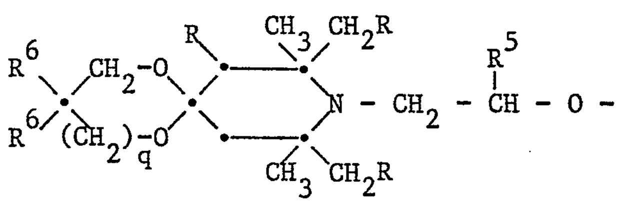

VIII

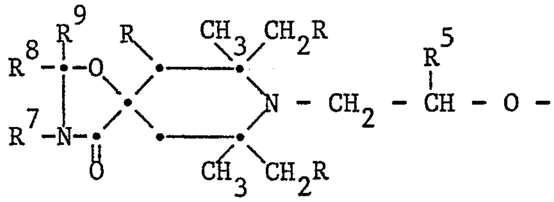

IX ,

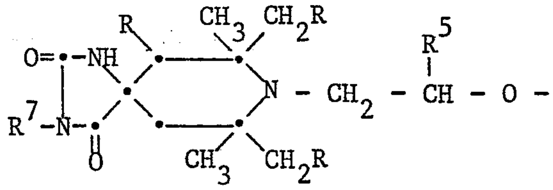

X ,

worin

R Wasserstoff oder $C_1$-$C_4$-Alkyl ist,

$R^1$ $C_1$-$C_{18}$-Alkyl, $C_3$-$C_{12}$-Alkenyl, $C_3$-$C_5$-Alkinyl, $C_7$-$C_{15}$-Aralkyl, $C_1$-$C_{18}$-Alkanoyl, $C_3$-$C_5$-Alkenoyl, Benzoyl, $C_3$-$C_{10}$-Alkoxycarbonylalkyl, Cyanomethyl oder Oxyl-Sauerstoff ist,

X -O-, -NH-, $-NR^2-$, $-OCH_2CH_2CH_2NH-$ oder $-CH_2CH_2NH-$ bedeutet, wobei $R^2$ $C_1$-$C_{18}$-Alkyl, $C_5$-$C_{12}$-Cycloalkyl oder -Alkylcycloalkyl, $C_7$-$C_{16}$-Aralkyl, $C_4$-$C_{15}$-Aryl oder Alkaryl, $C_3$-$C_{12}$-Mono-, Di- oder Trioxaalkyl, oder eine Gruppe der Formel

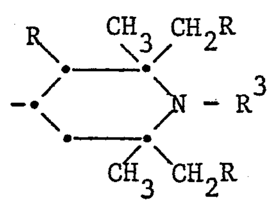

ist, wobei $R^3$ $C_1$-$C_{12}$-Alkyl, $C_3$-$C_5$-Alkenyl, $C_7$-$C_{12}$-Aralkyl, $C_1$-$C_{12}$-Alkanoyl oder Cyanomethyl darstellt,

$R^4$ $C_1$-$C_{18}$-Alkyl, $C_3$-$C_5$-Alkenyl, $C_3$-$C_5$-Alkinyl, $C_7$-$C_{12}$-Aralkyl, $C_1$-$C_{12}$-Alkanoyl, $C_3$-$C_5$-Alkenoyl, Benzoyl oder Cyanomethyl ist,

$R^5$ Wasserstoff, $C_1$-$C_4$-Alkyl, $C_1$-$C_4$-Alkoxy, Phenoxy, Tolyloxy oder Phenyl ist,

p 1 oder 2 und q 1 oder null ist,

r im Falle von q = 1 null oder 1 ist,
und im Falle von q = 0 die Zahl 1 ist,

$R^6$ H, Methyl oder Ethyl ist,

$R^7$ Wasserstoff, $C_1$-$C_{12}$-Alkyl, $C_3$-$C_5$-Alkenyl oder $C_7$-$C_{12}$-Aralkyl ist,

$R^8$ $C_2$-$C_{12}$-Alkyl, Cyclohexyl, Benzyl, Phenyl, Tolyl und

$R^9$ $C_1$-$C_{12}$-Alkyl ist, oder

$R^8$ und $R^9$ zusammen $C_4$-$C_{12}$-Alkylen sind,

D ein zweiwertiger Piperidinrest der Formel XI ist

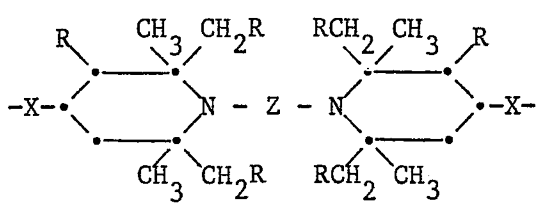

XI,

worin

Z $C_2$-$C_{12}$-Alkylen oder -Alkenylen, Xylylen oder eine Gruppe der Formel -CO-NH-B-NH-CO- oder $-CH_2-CH(R^5)-O-CO-R^{10}-CO-O-CH(R^5)-CH_2-$ bedeutet, worin $R^{10}$ $C_2$-$C_{12}$-Alkylen, $C_2$-$C_4$-Alkenylen, $C_5$-$C_7$-Cycloalkylen, $C_6$-$C_{12}$-Arylen oder -NH-B-NH- ist und

B den (m + 1)-wertigen Rest eines aliphatischen, cycloaliphatischen oder aromatischen Polyisocyanates mit 4-40 C-Atomen bedeutet.

Ein zweiter Weg zur Herstellung von isocyanatgruppenhaltigen Polyalkylpiperidinen, die monomer stabil sind, ist die Umsetzung von in 1-Stellung unsubstituierten Polyalkylpiperidinen mit Polyisocyanaten. Es war zu erwarten, dass eine solche N-Carbamoylierung des sterisch gehinderten Stickstoffatomes nur unter drastischen Bedingungen zu erzielen ist. Beispielsweise wird in der DE-OS 2,258,752, Beispiel 87, die Umsetzung mit Methylisocyanat 24 Stunden in siedendem Benzol durchgeführt. Auch in der DE-OS 27 19 132, Seite 26, wird empfohlen, die Umsetzung von Isocyanaten am sterisch gehinderten Piperidin-Stickstoff vorzugsweise bei 70-150°C durchzuführen. Es wurde jedoch überraschend gefunden, dass eine 1-Carbamoylierung sterisch gehinderter Piperidine bereits bei niedrigen Temperaturen abläuft, vorzugsweise bei -20° bis +30°C. Auf diese Weise sind nunmehr in 1-Stellung durch isocyanatgruppenhaltige Reste substituierte Polyalkylpiperidine erstmals zugänglich.

Gegenstand der Erfindung sind daher auch Verbindungen der Formel Ia und IIa,

$$A' - \overset{O}{\overset{\|}{C}} - NH - B - (NCO)_m \qquad Ia$$

$$(OCN)_m - B - NH - \overset{O}{\overset{\|}{C}} - D' - \overset{O}{\overset{\|}{C}} - NH - B - (NCO)_m \qquad IIa,$$

worin A' ein einwertiger Piperidinrest der Formel XII ist,

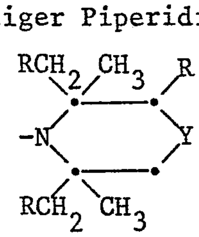

XII

und Y eine Gruppe 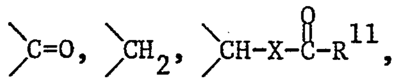,

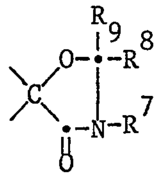, 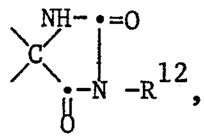, 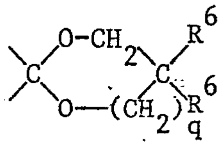

darstellt, worin $R^{11}$ $C_1$-$C_{18}$-Alkyl, $C_2$-$C_8$-Alkenyl, $C_7$-$C_{15}$-Aralkyl, $C_5$-$C_{12}$-Cycloalkyl, $C_6$-$C_{15}$-Aryl oder -Alkylaryl, $-NH-R^{13}$ oder $-N(R^{13})_2$ darstellt und $R^{13}$ $C_1$-$C_{12}$-Alkyl, Benzyl, Allyl, Cyclohexyl, $C_6$-$C_{15}$-Aryl oder -Alkaryl ist, $R^{12}$ Wasserstoff, $C_1$-$C_{12}$-Alkyl, $C_3$-$C_5$-Alkenyl oder $C_7$-$C_{12}$-Aralkyl ist,

D' ein zweiwertiger Piperidinrest der Formel XIII bis XV ist,

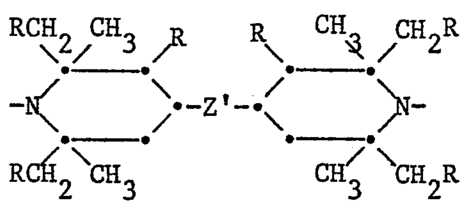

XIII

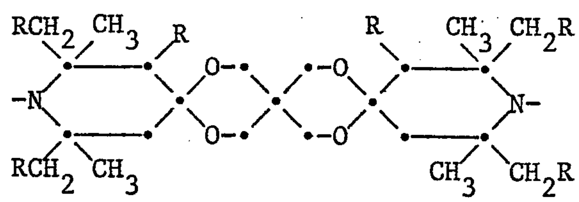

XIV

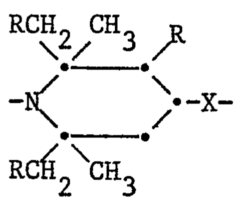

XV

worin

Z' einer der Reste $-N(R^{14})-$, $-N(R^{15})-CO-N(R^{15})-$, $-N(R^{15})-CO-CO-N(R^{15})-$, $-N(R^{15})-CO-R^{16}-CO-N(R^{15})-$, $C_4$-$C_{10}$-Alkylen, p-Xylylen,

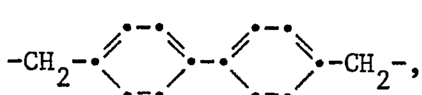

$-O-R^{17}-O-$, 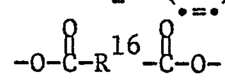 oder 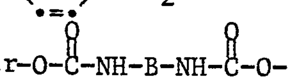 ist und

$R^{14}$ $C_1$-$C_{12}$-Alkyl, $C_3$-$C_5$-Alkenyl, Cyclohexyl oder $C_2$-$C_8$-Alkanoyl,

$R^{15}$ Wasserstoff, $C_1$-$C_{12}$-Alkyl, Cyclohexyl oder $C_3$-$C_8$-Alkoxyalkyl,

$R^{16}$ $C_2$-$C_{12}$-Alkylen, $C_2$-$C_4$-Alkenylen, $C_5$-$C_7$-Cycloalkylen oder $C_6$-$C_{12}$-Arylen und

$R^{17}$ $C_2$-$C_{12}$-Alkylen, 2-Butenylen-1,4 oder Xylylen bedeuten, und m, B und R die für Formel I gegebene Bedeutung haben.

In diesen Formeln können die Reste R, $R^1$, $R^2$, $R^3$, $R^5$, $R^7$, $R^8$, $R^9$, $R^{11}$, $R^{12}$, $R^{14}$ und $R^{15}$ Alkylreste sein. Diese können, im Rahmen der definierten C-Anzahl, beispielsweise Methyl, Ethyl, Propyl, Isopropyl, n-Butyl, tert.Butyl, Isoamyl, n-Hexyl, 2-Ethylhexyl, n-Octyl, n-Decyl, n-Dodecyl, n-Tetradecyl, n-Hexadecyl oder n-Octadecyl sein. $R^2$ als Oxaalkyl kann z.B. 3-Oxabutyl, 3-Oxaheptyl, 3,6-Dioxaheptyl oder 3,6,9-Trioxanonyl sein. $R^{15}$ als Alkoxyalkyl kann z.B. 2-Methoxyethyl, 2-Butoxyethyl oder 3-Ethoxypropyl sein.

Die Reste $R^1$, $R^2$, $R^4$, $R^7$ und $R^{12}$ als Alkenyl können - im Rahmen der definierten C-Anzahl - z.B. Allyl, Methallyl, 1,1-Dimethylallyl oder 3-Butylallyl sein. $R^{11}$ als Alkenyl kann z.B. Vinyl, 2-Propenyl oder 2,2-Dimethylvinyl sein. $R^1$, $R^2$ und $R^4$ als Alkinyl können z.B. Propargyl oder 2-Butinyl sein.

$R^1$, $R^2$, $R^3$, $R^7$, $R^{11}$ und $R^{12}$ als Aralkyl können z.B. Benzyl, α-Methylbenzyl, α,α-Dimethylbenzyl, 2-Phenylethyl oder 3-Phenylpropyl sein.

$R^2$, $R^4$ und $R^{11}$ als Cycloalkyl oder Alkylcycloalkyl können z.B. Cyclopentyl, Cyclohexyl, Methylcyclohexyl, Dimethylcyclohexyl, Cyclooctyl oder Cyclododecyl sein.

$R^2$, $R^{11}$ und $R^{13}$ als Aryl oder Alkylaryl können z.B. Phenyl, Tolyl, Xylyl, 3-Propylphenyl, 4-tert.Butylphenyl, 4-Nonylphenyl oder Naphthyl sein.

$R^1$, $R^3$ und $R^4$ als Alkanoyl können, im Rahmen der definierten C-Anzahl, z.B. Formyl, Acetyl, Propionyl, Isobutyroyl, 2-Ethyl-Capryl, Octanoyl, Decanoyl, Lauroyl oder Stearoyl sein. $R^{14}$ als Alkanoyl kann z.B. Acetyl, Propionyl, Butyryl oder Isobutyroyl sein.

$R^1$ und $R^4$ als Alkenoyl können z.B. Acryloyl, Methacryloyl oder Crotonoyl sein. $R^1$ als Alkoxycarbonylalkyl kann z.B. Butoxycarbonylmethyl, Methoxycarbonylethyl oder Ethoxycarbonylethyl sein.

Z, $R^{10}$, $R^{16}$ und $R^{17}$ als Alkylen können z.B. 1,2-Ethylen, 1,2-Propylen, 1,3-Propylen, 1,4-Butylen, 2,2-Dimethyl-1,3-propylen, Hexamethylen, Octamethylen, Decamethylen oder Dodecamethylen sein. $R^8$ und $R^9$ können zusammen Alkylen mit 4-12 C-Atomen sein und bilden dann zusammen mit dem C-Atom, an das sie gebunden sind, einen Cycloalkanring, z.B. einen Cyclopentan-, Cyclohexan-, Methylcyclohexan-, Cyclooctan- oder Cyclododecanring.

$R^{10}$ und $R^{16}$ als Alkenylen kann z.B. Vinylen, Propenylen-1,2 oder 2-Butenylen-1,4 sein.

$R^{10}$ und $R^{16}$ als Cycloalkylen können z.B. 1,3-Cyclopentylen, 1,3-Cyclohexylen oder 1,4-Cyclohexylen sein. $R^{10}$ und $R^{16}$ als Arylen können z.B. 1,3- oder 1,4-Phenylen, 4,4'-Diphenylenmethan oder 4,4'-Diphenylen sein.

B als Rest eines Polycyanates kann ein 2-, 3- oder 4-wertiger aliphatischer, cycloaliphatischer oder aromatischer Rest sein. Beispiele für 2-wertige Reste sind Tetra-, Hexa-, Octa-, Dodecamethylen, 2,2,4- oder 2,4,4-Trimethylhexamethylen, Tolylen-2,4, Tolylen-2,6, Naphthylen-1,5 oder ein Rest der Formeln

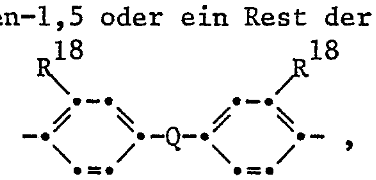 , 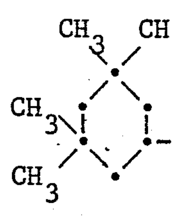 oder 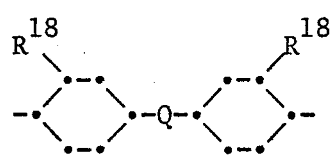

worin Q eine direkte Bindung, $-CH_2-$ oder $(CH_3)_2C<$ bedeuten und $R^{18}$ H oder $CH_3$ ist. Beispiele für dreiwertige Reste sind die Reste der folgenden Formeln

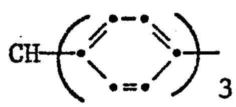 , 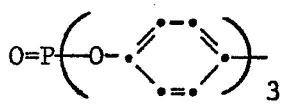 und 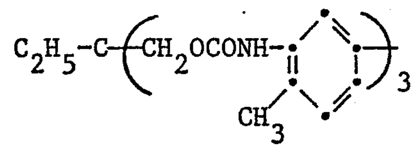

Beispiele für vierwertige Reste sind Reste der Formel

$$C\left(CH_2OCONH-R^{19}\right)_4 ,$$

worin $R^{19}$ ein zweiwertiger Rest B ist.

Unter diesen Verbindungen der Formel I, II, Ia und IIa sind jeweils die Verbindungen bevorzugt, worin m 1 ist und B ein zweiwertiger Rest ist. Ausserdem sind die Verbindungen der Formeln I und Ia bevorzugt gegenüber den Verbindungen der Formeln II und IIa.

Besonders bevorzugt sind die Verbindungen der Formel I, worin m 1 ist, B ein zweiwertiger Rest ist und A ein Piperidinrest der Formel III ist, in dem R Wasserstoff bedeutet. Unter diesen Verbindungen sind wiederum solche bevorzugt, worin $R^1$ $C_2$-$C_{12}$-Alkyl, $C_3$-$C_5$-Alkenyl, Benzyl, $C_2$-$C_4$-Alkanoyl oder Benzoyl bedeutet, insbesondere solche, worin $R^1$ $C_1$-$C_4$-Alkyl, Allyl, Benzyl oder Acetyl bedeutet.

Beispiele für Verbindungen der Formel I sind die Verbindungen der folgenden Formel, wobei das Symbol

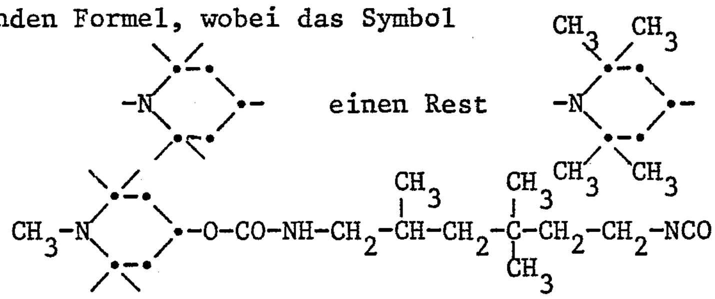

einen Rest bedeutet.

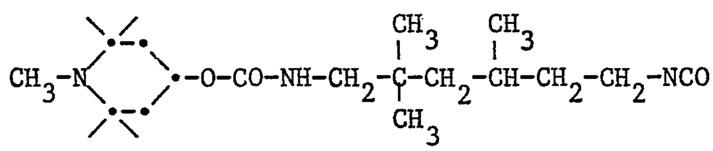

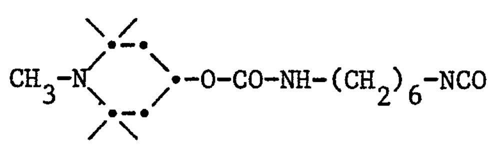

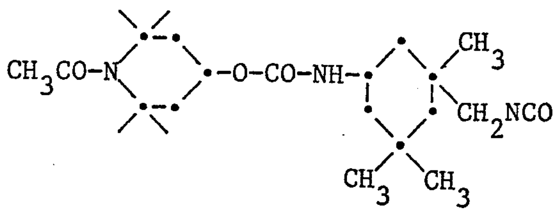

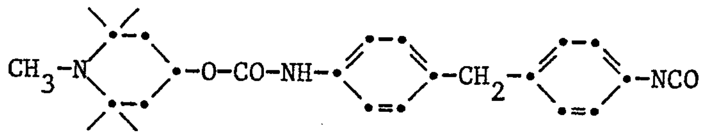

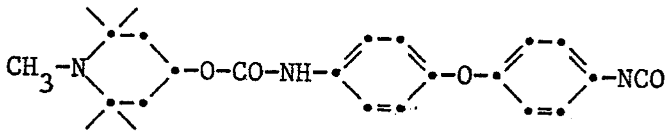

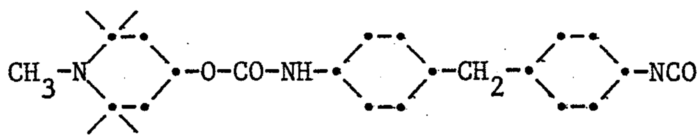

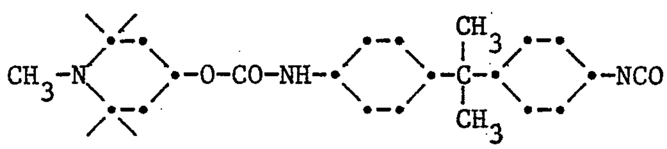

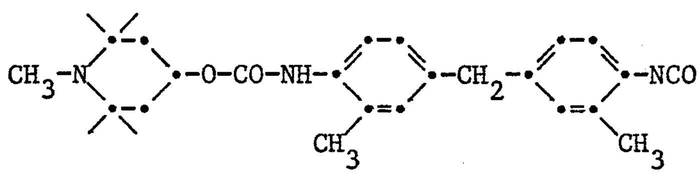

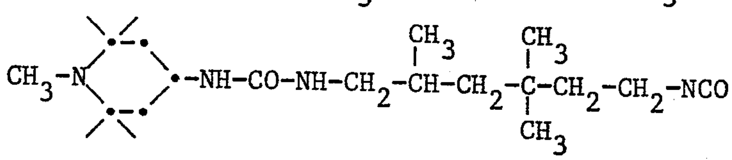

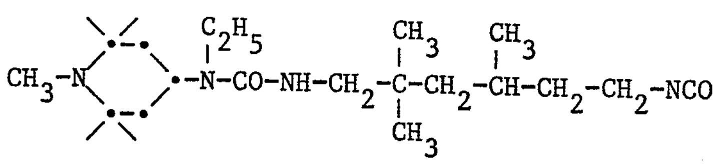

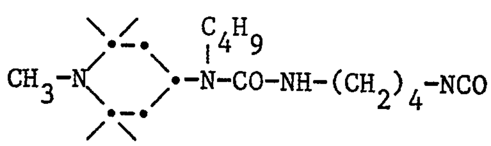

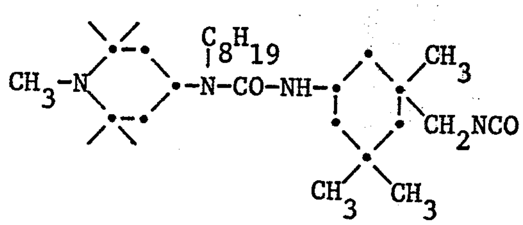

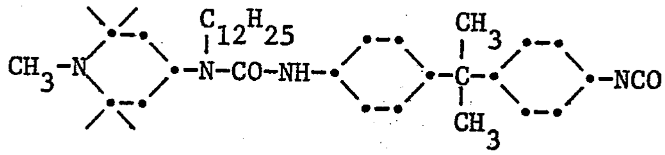

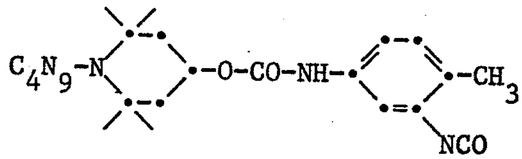

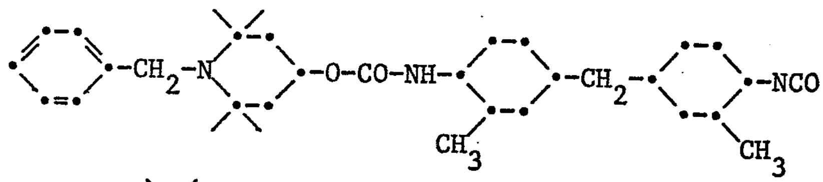

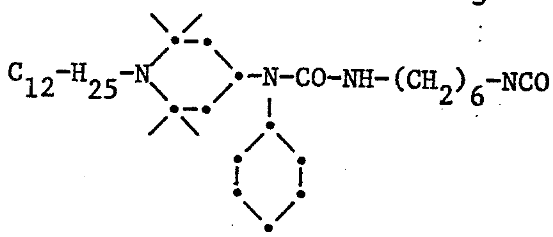

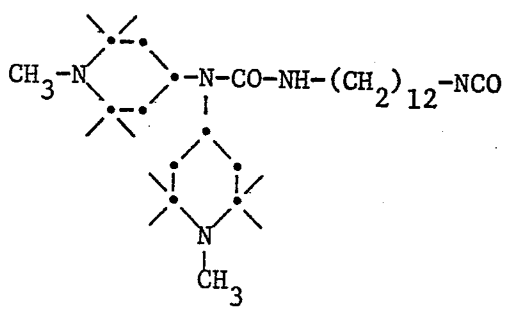

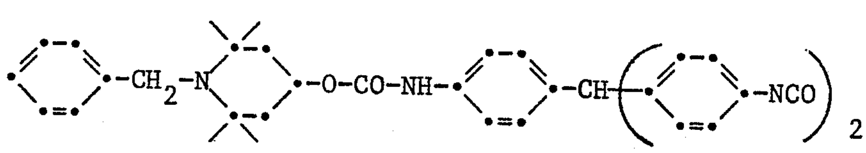

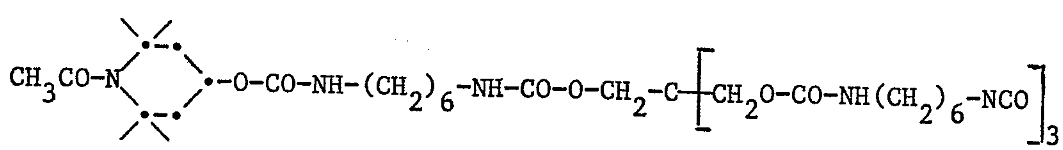

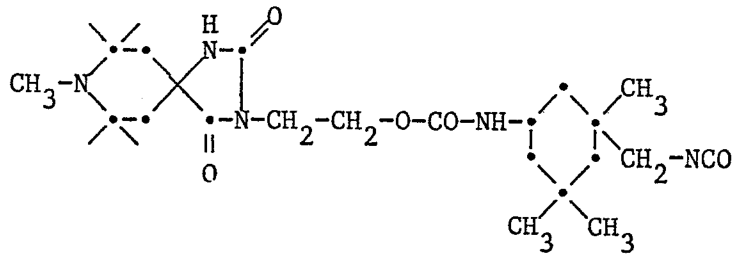

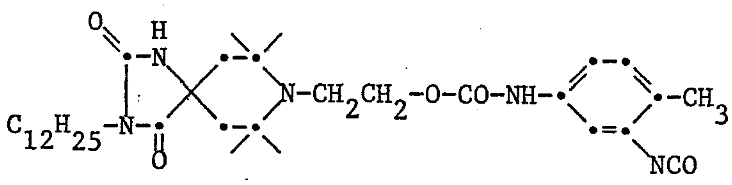

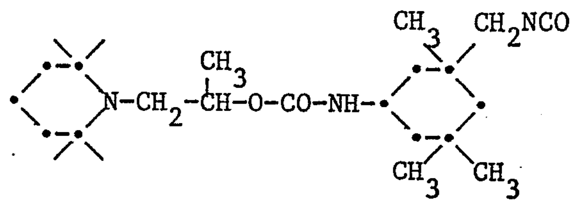

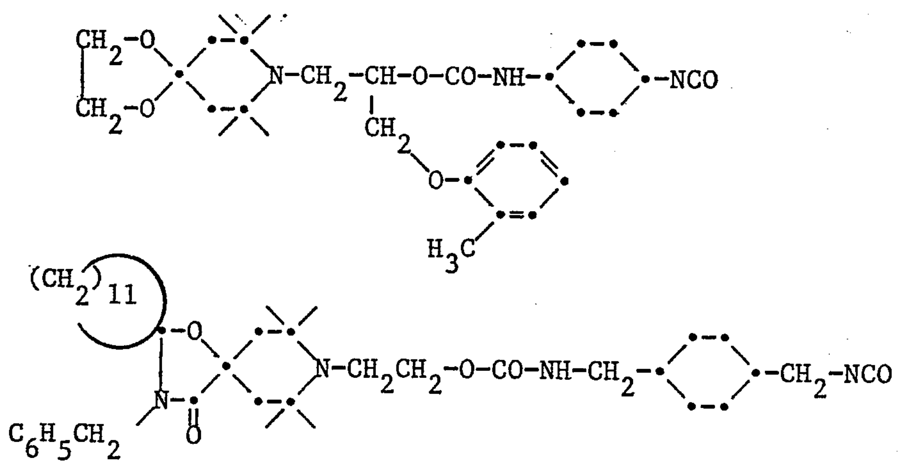

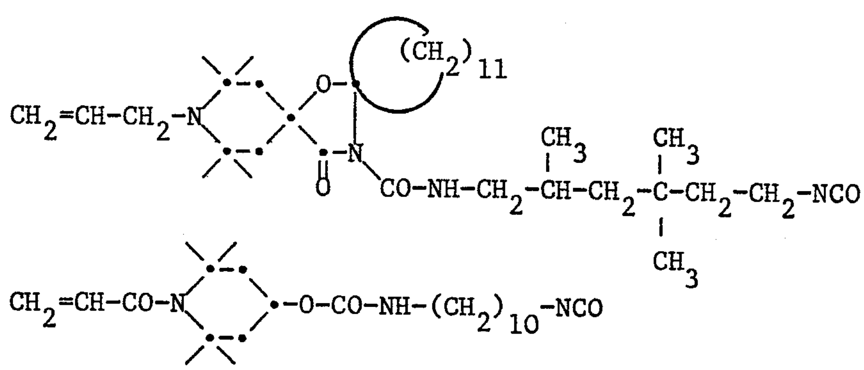

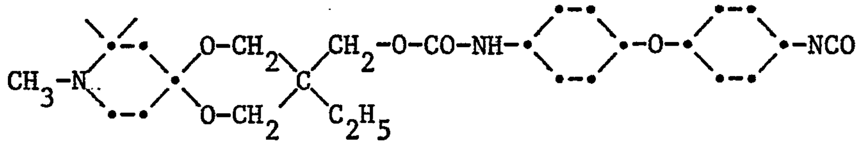

Beispiele der Formel II sind die Verbindungen der folgenden Formeln:

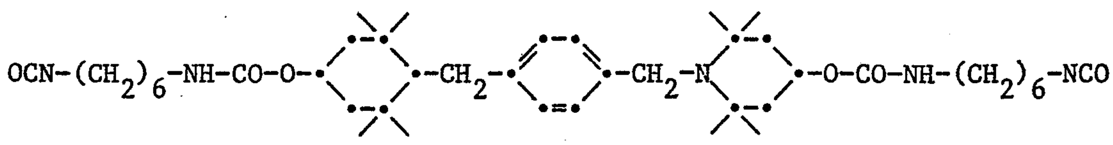

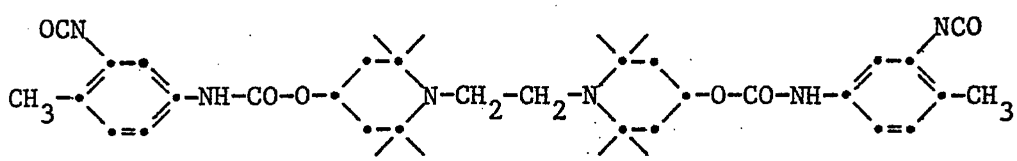

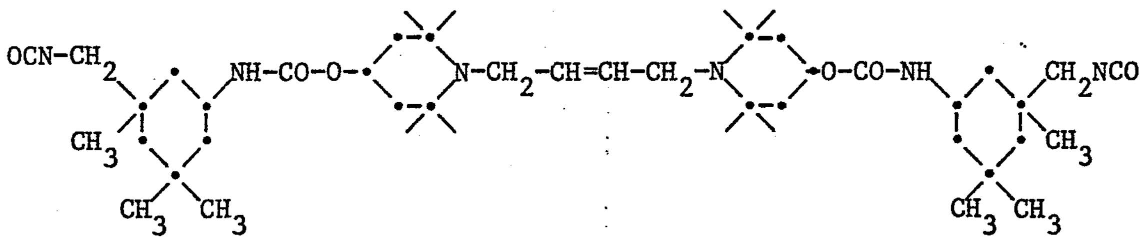

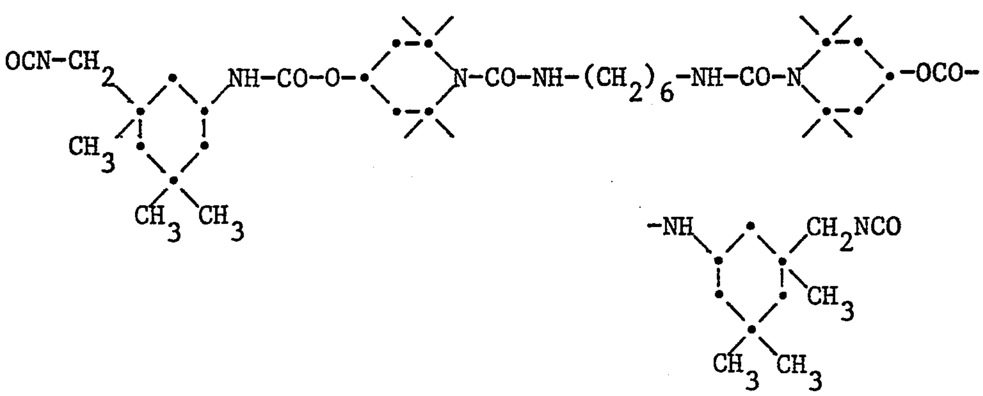

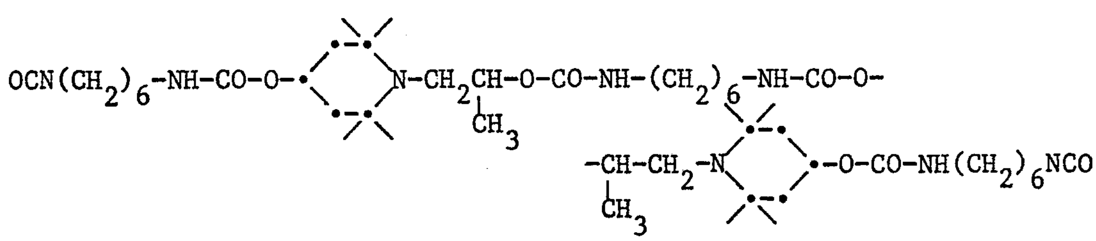

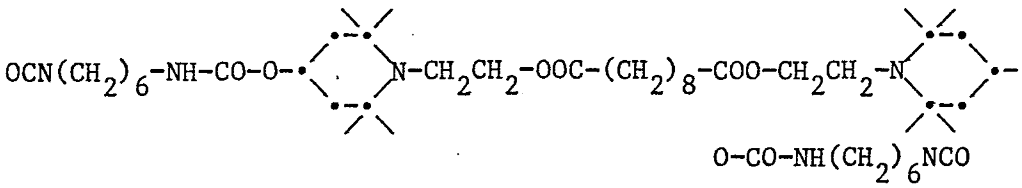

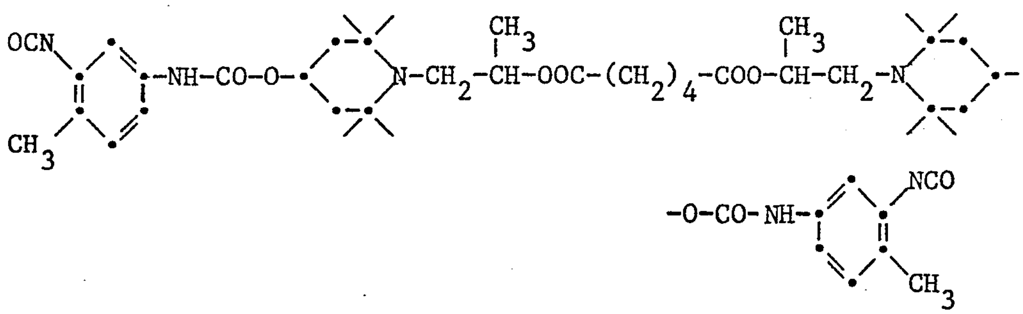

Beispiele für Verbindungen der Formel Ia sind folgende Verbindungen:

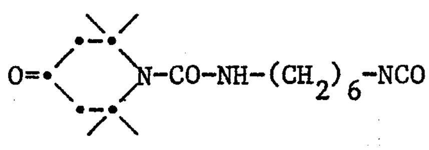

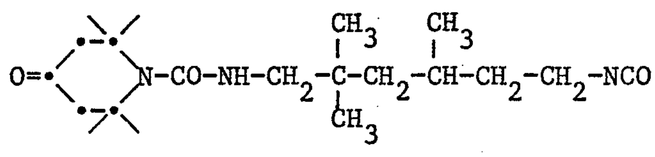

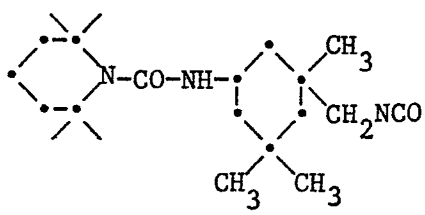

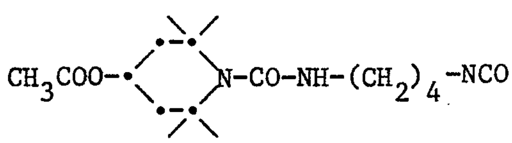

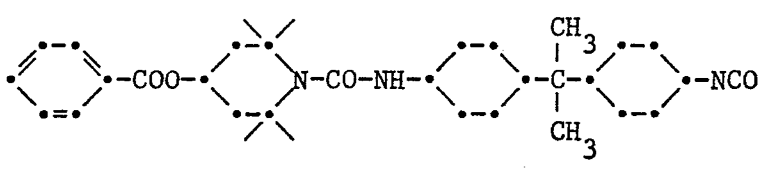

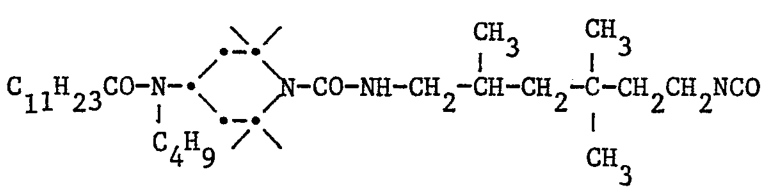

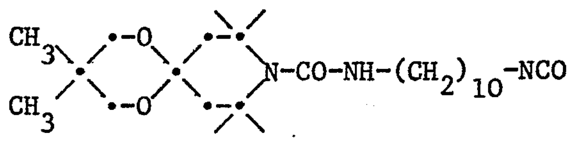

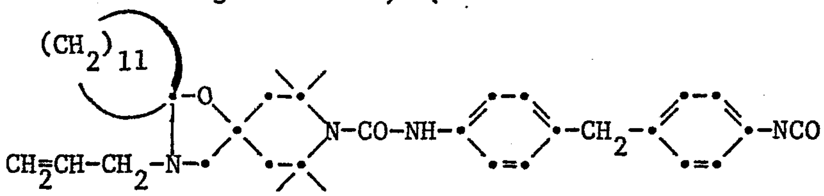

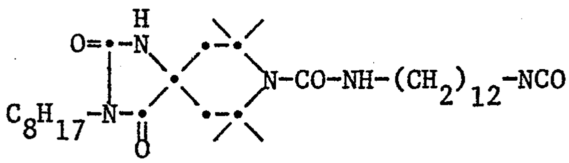

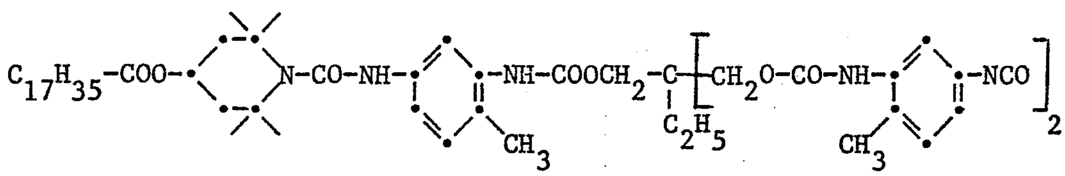

Beispiele für Verbindungen der Formel IIa sind die folgenden Verbindungen:

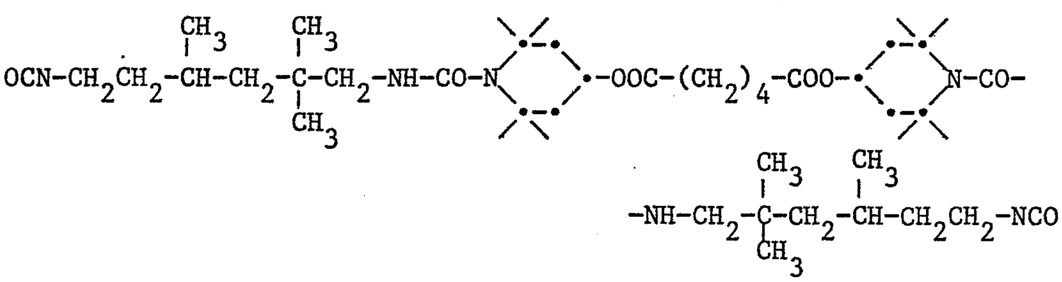

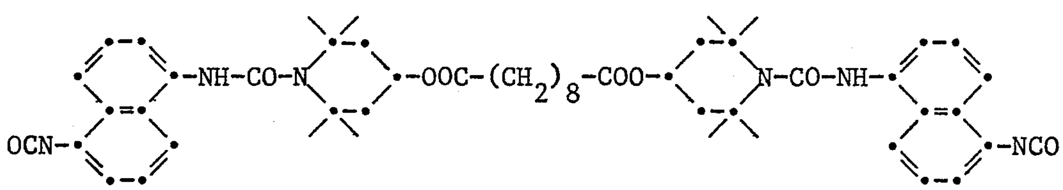

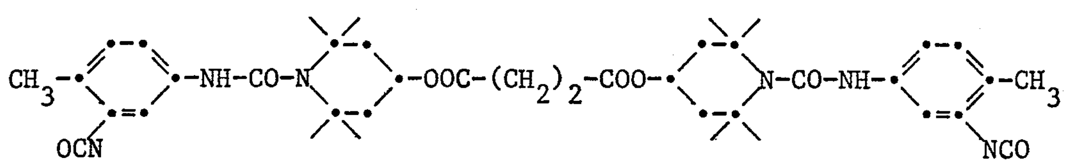

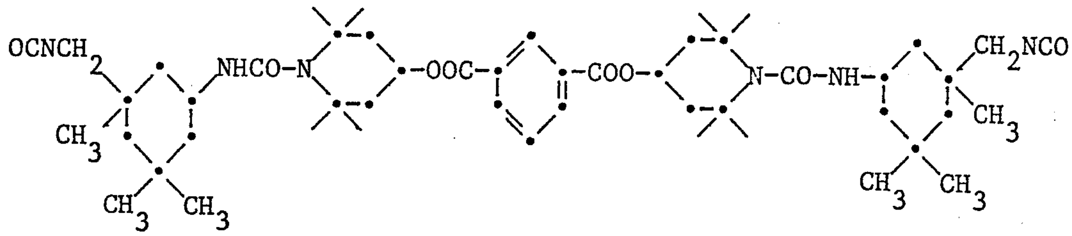

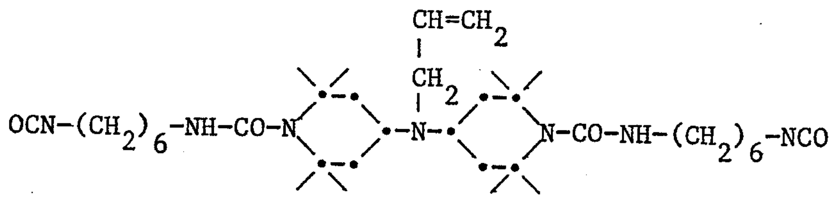

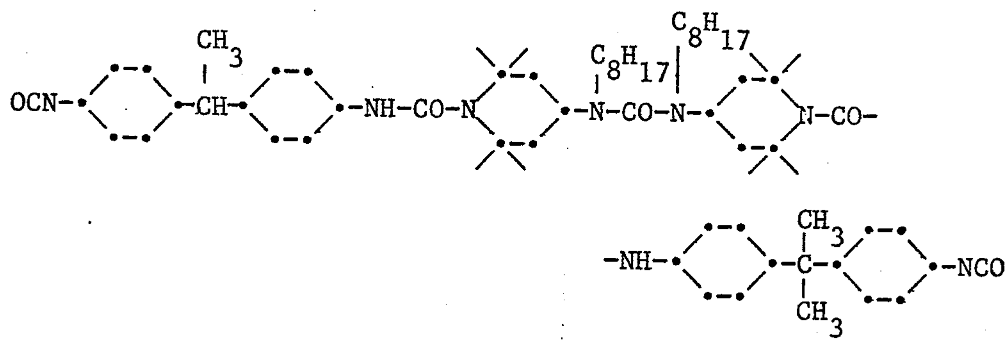

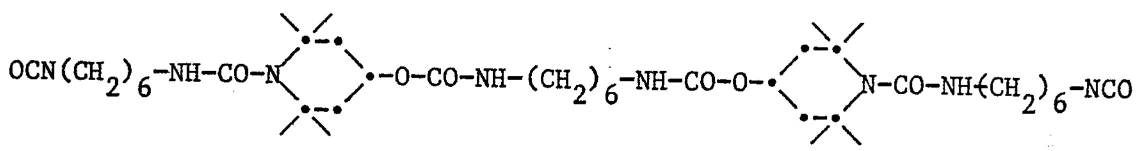

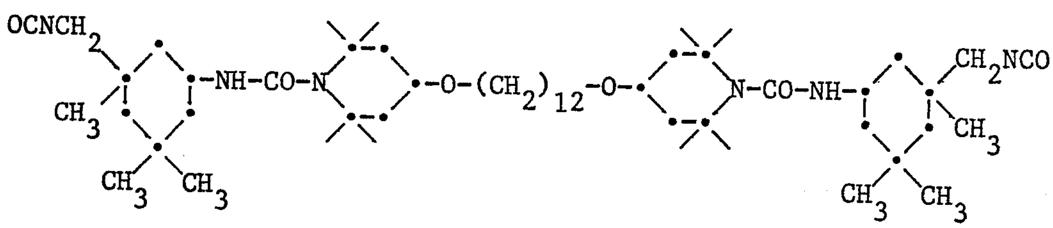

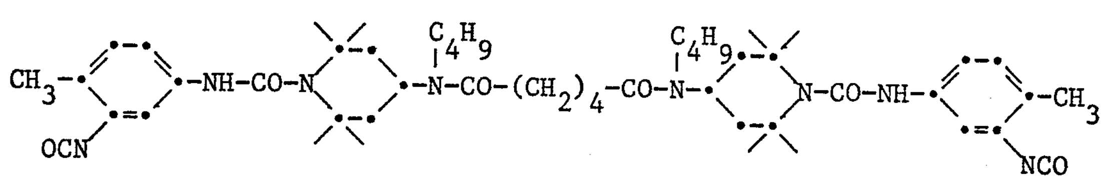

Die Herstellung der Verbindungen der Formel I erfolgt durch Umsetzung einer Verbindung AH mit mindestens einem Mol und die Herstellung der Verbindungen der Formel II durch Umsetzung einer Verbindung H-D-H mit mindestens 2 Mol eines Polyisocyanates B $(NCO)_{m+1}$, in beiden Fällen

jedoch vorzugsweise mit einem Ueberschuss des Polyisocyantes und vorzugsweise in einem inerten organischen Lösungsmittel. Als Lösungsmittel eignen sich beispielsweise Kohlenwasserstoffe wie Toluol, Xylol, Hexan, Heptan, Aliphatengemische oder Cyclohexan, oder Ether wie Diethylether, Diisopropylether, Ethylenglykoldimethylether, Tetrahydrofuran oder Dioxan. Der verwendete Ueberschuss an Polyisocyanat kann ein mehrfaches der stöchiometrischen Menge betragen. Besitzt das Polyisocyanat eine bevorzugt reagierende Isocyanatgruppe, so benötigt man einen geringeren Ueberschuss als bei Polyisocyanaten mit Isocyanatgruppen gleicher Reaktivität. Zweckmässig legt man das Polyisocyanat als Lösung vor und gibt das Piperidinderivat AH oder HDH, ebenfalls in gelöster Form, langsam unter Rühren zu. Die Reaktion kann durch Zusatz katalytischer Mengen einer starken organischen Base, wie z.B. 1,4-Diazabicyclo[2.2.2]-octan, 1,8-Diazabicyclo[5.4.0]undec-7-en oder Benzyltrimethylammoniumhydroxid, beschleunigt werden. Man kann die Reaktion durch leichtes Erwärmen beschleunigen und destilliert anschliessend das Lösungsmittel und den Ueberschuss des Polyisocyanates ab. Die als Rückstand hinterbleibenden Produkte der Formel I oder II haben überraschenderweise trotz Vorliegens einer -CO-NH-Gruppe keine ausgeprägte Kristallisationstendenz. Sie fallen teilweise als amorphe Massen oder Harze an. Die Analyse dieser anfallenden Rohprodukte zeigt, dass sie weitgehend aus dem monomeren Polyisocyanat der Formel I oder II bestehen.

Die Verbindungen der Formel Ia und IIa werden, wie vorhin erwähnt, durch Umsetzung von Verbindungen A'-H oder H-D'-H mit den Polyisocyanaten $B(NCO)_{m+1}$ bei niedrigen Temperaturen, vorzugsweise bei -20° bis +30°C, hergestellt. Auch hierbei arbeitet man vorzugsweise in Lösung und verwendet einen Ueberschuss an $B(NCO)_{m+1}$, den man nach Ende der Umsetzung zurückgewinnen kann. Die hierfür verwendbaren Lösungsmittel sind dieselben wie oben erwähnt.

Wie bei allen Reaktionen mit Isocyanaten ist es auch bei der Herstellung der Verbindungen der Formeln I, II, Ia und IIa wichtig,

dass die verwendeten Lösungsmittel wasserfrei sind und dass man alle Operationen unter trockenen Inertgasen ausführt.

Die Verbindungen der Formeln I, II, Ia und IIa sind Lichtschutzmittel für organische Materialien, insbesondere für organische Polymere. Beispiele für Polymere, die durch Lichteinwirkung normalerweise geschädigt werden und durch Zusatz der erfindungsgemässen Verbindungen stabilisiert werden können, sind Polyolefine, Polydiene, Polystyrole, Polyvinylhalogenide, -alkohole, -carboxylate, Poly(meth)acrylsäurederivate, Polyether, Polyester, Polyamide, Polycarbonate, Polyurethane, Polyharnstoffe, Epoxidharze, Alkydharze, Polyesterharze, Acrylharze, Phenolharze, Melaminharze und deren Copolymeren sowie natürliche Polymere wie Cellulose, Cellulosederivate oder Gelatine.

Von besonderer Bedeutung sind solche Polymere, die funktionelle Gruppen besitzen, die mit Isocyanatgruppen reagieren können, beispielsweise Hydroxyl-, Amino- oder Carboxylgruppen. Beispiele für solche Polymere sind Polyvinylalkohol und dessen Copolymerisate, Cellulose und deren partielle Ether und Ester, (Meth)acrylsäurecopolymerisate, partielle Poly(meth)acrylsäureester von Polyolen, Poly(meth)acrylsäureamide von Alkanolaminen, hydroxylgruppenhaltige Polyester, Polyether und Alkydharze, Polyurethane und Polyharnstoffe mit reaktiven Endgruppen, Epoxidharze, Phenolharze, Melaminharze und Gelatine.

Die Verbindungen der Formel I und Ia, in denen m = 1 ist, haben nur eine Isocyanatgruppe, mit der sie an die polymere Matrix gebunden werden können. Alle anderen Verbindungen der Formel I und Ia sowie die Verbindungen der Formel II und IIa haben mehr als eine Isocyanatgruppe. Dadurch können sie mit dem funktionellen Polymeren unter Vernetzung reagieren. Dies ist z.B. bei Lackharzen erwünscht, die nach dem Aufbringen in eine unlösliche Form überführt werden sollen. In solchen Fällen kann der Stabilisator gleichzeitig auch als Härter fungieren.

Verbindungen der Formel I und Ia, in denen m = 2 ist, und Verbindungen der Formel II und IIa, in denen m = 1 ist, sind Diisocyanate. Diese können auch zum Aufbau von linearen Polyurethanen verwendet werden. Verwendet man sie als einziges Diisocyanat oder als vorwiegendes Diisocyanat, so erhält man Polyurethane, die reich an Polyalkylpiperidingruppen sind und ihrerseits als polymere Stabilisatoren für Polymere verwendet werden können, wie dies in der DE-OS 27 19 131 beschrieben ist.

Verwendet man die erfindungsgemässen Diisocyanate in relativ kleinen Mengen neben anderen Diisocyanaten, so erhält man stabilisierte Polyurethane, die ein eingebautes Lichtschutzmittel in der Polymerkette enthalten, wie dies in der DE-OS 27 19 132 beschrieben ist. Diese linearen Polyurethane können durch Mitverwendung von tri- oder höherfunktionellen Komponenten auch in vernetzte Polyurethane übergeführt werden, wie sie z.B. für Schaumstoffe oder Lacke Verwendung finden.

Je nach dem verschiedenen Verwendungszweck wird man also die erfindungsgemässen Isocyanate entweder dem Polymeren vor dessen formgebender Verarbeitung zusetzen oder man setzt sie den Monomeren während der Herstellung des Polymeren zu. Bei der Verwendung in Lacken sind es meist Prepolymere mit funktionellen Gruppen, denen man das erfindungsgemässe Isocyanat vor der Applikation und Härtung zusetzt. In Mehrschichtlacken setzt man die erfindungsgemässen Isocyanate vor allem dem Decklack zu.

Mit den verschiedenen Einsatzgebieten schwankt auch die Menge des zugesetzten Isocyanates stark. Setzt man es nur als Lichtschutzmittel zu, so genügen 0,05 bis 5%, vorzugsweise 0,1 bis 1%, bezogen auf das zu stabilisierende Polymere.

Dabei kann man noch andere Stabilisatoren oder sonstige in der Kunststoff- und Lacktechnologie übliche Zusätze mitverwenden. Beispiele hierfür sind andere Lichtschutzmittel, Antioxidantien, Metalldesaktivatoren, Thiosynergisten, Phosphat-Costabilisatoren, Metallcarboxylate, Füllstoffe, Pigmente, Glasfasern, Flammschutzmittel, Antistatika, Verlaufshilfsmittel oder Thixotropiemittel. Beispiele für einzelne solcher bekannter Additive sind in der DE-OS 24 27 853 auf Seite 18-24 beschrieben.

Von besonderer Bedeutung ist die Mitverwendung von Lichtschutzmitteln, welche UV-Absorber sind, wie z.B. von Derivaten des 2-(2-Hydroxyphenyl)-benztriazols, des 2-Hydroxybenzophenons, des Oxalsäuredianilides, der α-Cyanacrylsäure oder der α-Cyanzimtsäure. Hierbei kommt es meist zu einer synergistischen Steigerung der Lichtschutzwirkung, insbesondere in Lackharzen.

Die folgenden Beispiele beschreiben einige der erfindungsgemässen Verbindungen, ihre Herstellung und ihre Verwendung. Dabei sind die Temperaturen als °C angegeben, Teile bedeuten Gewichtsteile.

Beispiel 1: Herstellung von Isocyanaten der Formel I und II.

Zu einer Lösung von 34,8 g (0,2 Mol) 2,4-Toluylendiisocyanat und 0,1 g 1,4-Diazabicyclo[2.2.2]octan (DABCO) in 100 ml wasserfreiem Tetrahydrofuran werden bei 50° unter Rühren innerhalb von 5 h 19,9 g (0,1 Mol) 1-Acetyl-4-hydroxy-2,2,6,6-tetramethylpiperidin, gelöst in 100 ml Tetrahydrofuran, getropft und anschliessend 16 h bei 50° weitergerührt. Hierauf wird im Wasserstrahlvakuum (unter Feuchtigkeitsausschluss) das Lösungsmittel und anschliessend im Hochvakuum bei ca. 100° und 0,03 mbar durch Dünnschichtdestillation das nicht umgesetzte überschüssige 2,4-Toluylendiisocyanat vom Reaktionsprodukt entfernt. Das so erhaltene 2-Methyl-5-(1-acetyl-2,2,6,6-tetramethylpiperidin-4-yloxy-carbonylamino)-phenylisocyanat (Verbindung Nr. 1) wird 4 h mit 200 ml wasserfreiem Diethylether (unter $N_2$) verrührt, das ausgefallene farblose Produkt unter $N_2$ abfiltriert und 3 h im Hochvakuum bei Raumtemperatur getrocknet. Das so erhaltene pulverige Monoisocyanat 1, mit einem Erweichungspunkt ($T_s$) von ca. 160°, besitzt einen NCO-Gehalt von 10,6 Gew.% (ber. 11,7%).
Das Infrarotspektrum zeigt deutlich die Anwesenheit der Isocyanatbande bei ∿ 2260 $cm^{-1}$ und das $^{13}C$-Resonanzspektrum steht mit der angegebenen Struktur in Einklang.

Analog wurden die nachfolgend aufgeführten Verbindungen hergestellt:

| Verbindung Nr. | Strukturformel | Physikal. Daten | Analyse NCO |
|---|---|---|---|
| 1 | 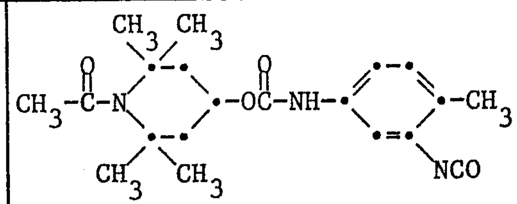 | $T_s$ ~160° | 10,6% |
| 2 | 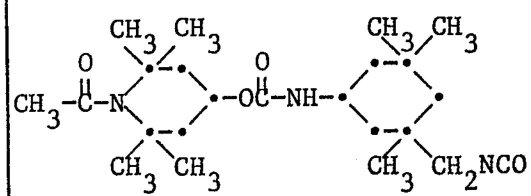 | viskose Masse | 8,9% |
| 3 | 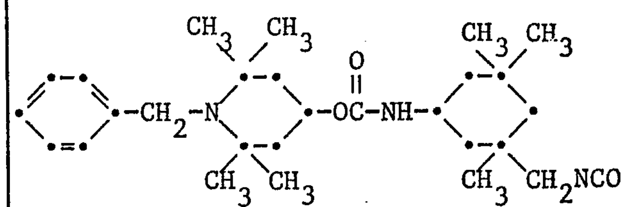 | viskose Masse | 8,2% |
| 4 | 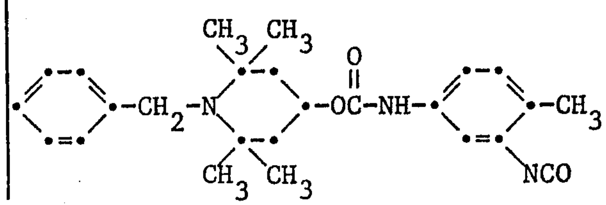 | viskose Masse | 9,3% |

| Verbindung Nr. | Strukturformel | Physikal. Daten | Analyse NCO |
|---|---|---|---|
| 5 | 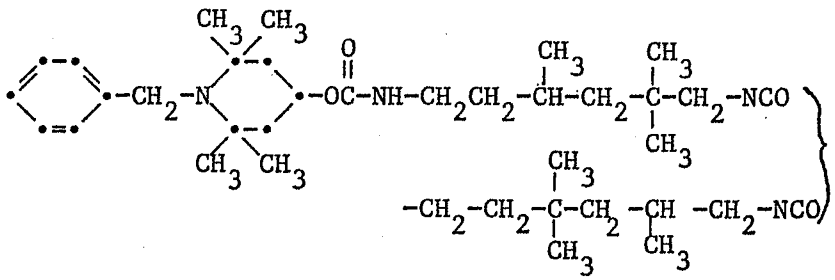 Gemisch | viskose Masse | 8,6% |
| 6 | 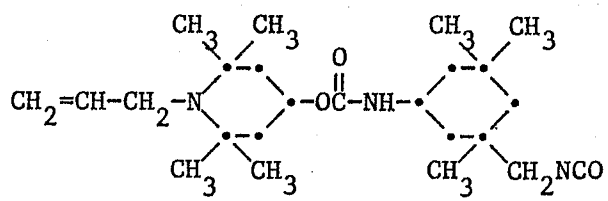 | $T_s$ ~60° | 9,0% |
| 7 | 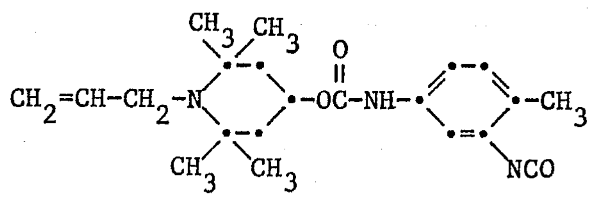 | viskose Masse | 9,9% |

| Verbindung Nr. | Strukturformel | Physikal. Daten | Analyse NCO |
|---|---|---|---|
| 8 | 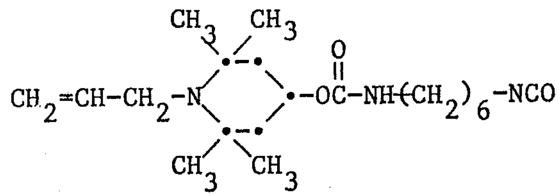 | viskose Masse | 10,2% |
| 9 | 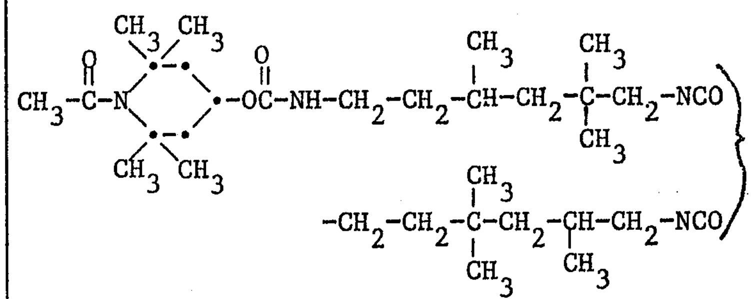 Gemisch | viskose Masse | 8,6% |
| 10 | 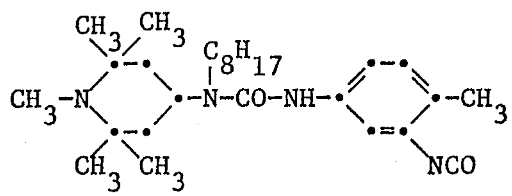 | $T_s \sim 55°$ | 8,8% |
| 11 | 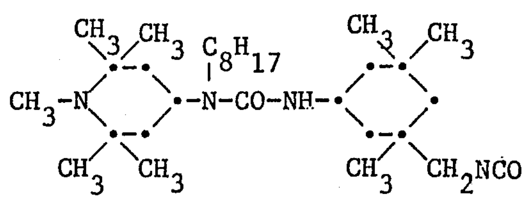 | $T_s \sim 30°$ | 7,6% |

| Verbindung Nr. | Strukturformel | Physikal. Daten | Analyse NCO |
| --- | --- | --- | --- |
| 12 | 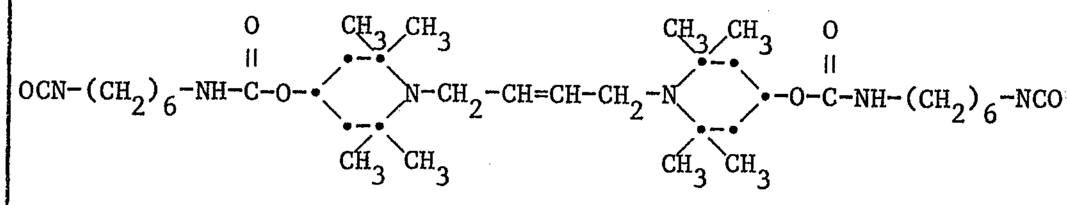 | Smp. 153-156° | 10,9 % |
| 13 | 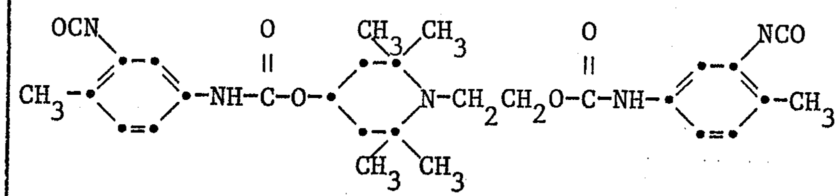 | $T_s$~100° | 14,3 % |

<u>Beispiel 2</u>: Herstellung von Isocyanaten der Formeln Ia und IIa

Zu einer Lösung von 13,07 g (0,05 Mol) 4-Benzoyloxy-2,2,4,4-tetramethylpiperidin in 100 ml wasserfreiem Diethylether tropft man unter Rühren be ca. 20° innerhalb von 3 Stunden die Lösung von 8,71 g (0,05 Mol) 2,4-Toluylen-diisocyanat in 100 ml Diethylether und rührt für weitere 20 Stunden bei Raumtemperatur. Anschliessend wird das Lösungsmittel im Vakuum (ca. 30 mbar) unter Feuchtigkeitsausschluss abdestilliert und der Rückstand im Hochvakuum (ca. 0,02 mbar) bei Raumtemperatur vollständig getrocknet.

Das so erhaltene pulverförmige, farblose Monoisocyanat der Formel

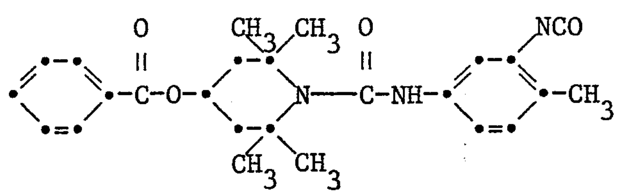

(Verbindung Nr. 14)

erweicht bei 70° und hat einen NCO-Gehalt von 11,4 % (Theorie 9,7 %).

Analog wurde das Diisocyanat der Formel

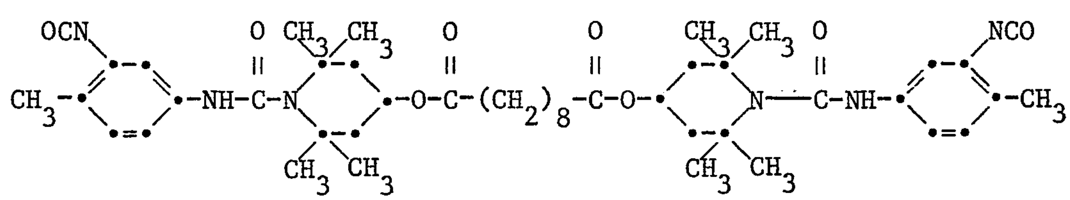

(Verbindung Nr. 15)

hergestellt, das bei 70° erweicht und einen NCO-Gehalt von 11,9 % (Theorie 10,1 %) aufweist.

Beispiel 3: Stabilisierung eines 2-Schicht-Metalleffekt-Lackes

Aluminiumbleche von 0,5 mm Stärke werden mit einem Aluminium-pigmentierten Grundlack auf Basis Polyester/Celluloseacetobutyrat/Melaminharz beschichtet. Auf den nassen Grundlack wird ein Klarlack der folgenden Zusammensetzung aufgespritzt:

| | |
|---|---|
| 58,3 Teile | Viacryl® VC 373 (Acrylharz der Fa. Vianova, Wien) |
| 27,3 Teile | Maprenyl® MF 590 (Melaminharz der Fa. Höchst AG, Frankfurt) |
| 1,0 Teil | 1 %ige Lösung eines Silikonharzes in Xylol |
| 4,0 Teile | Solvesso® 150 (aromatisches Lösungsmittelgemisch |
| 5,4 Teile | Xylol |
| 4,0 Teile | Ethylglykolacetat. |

Dazu kommen 0,85 Teile der in der folgenden Tabelle aufgeführten Isocyanate, was einem Gehalt von 1 % bezogen auf das Lackharz entspricht. Der Klarlack hat eine Viskosität von 21 sec/Din-Becher 4. Er wird in einer Schichtdicke von 40 µm aufgetragen und bei 130°C 39 Minuten eingebrannt.

Die Proben werden in einen UVCON - Schnellbewitterungsgerät der Fa. Atlas mit einem Zyklus von 4 h UV-Bestrahlung bei 60° und 4 h Bewitterung bei 50° 2000 h bewittert. Nach 1000 h und nach 2000 h wird der 20°-Glanz gemäss DIN 67530 gemessen. Ausserdem werden die Proben in regelmässigen Abständen unter dem Stereomikroskop auf Rissbildung untersucht.

| Lichtschutzmittel | 20° Glanz nach | | | Rissbildung |
|---|---|---|---|---|
| | 0 h | 1000 h | 2000 h | |
| keines | 94 | 66 | 6 | nach 1600 h |
| 1 % Verbindung Nr. 5 | 95 | 81 | 63 | keine |
| 1 % Verbindung Nr. 7 | 95 | 86 | 52 | keine |
| 1 % Verbindung Nr. 10 | 96 | 79 | 33 | keine |
| 1 % Verbindung Nr. 11 | 94 | 81 | 40 | keine |

Patentansprüche

1. Verbindungen der Formel I oder II

$$A - \overset{\overset{\displaystyle O}{\|}}{C} - NH - B - (NCO)_m \qquad I$$

$$(OCN)_m - B - NH - \overset{\overset{\displaystyle O}{\|}}{C} - D - \overset{\overset{\displaystyle O}{\|}}{C} - NH - B - (NCO)_m \qquad II ,$$

worin m 1 bis 3 ist,

A ein einwertiger Piperidinrest der Formeln III bis X ist,

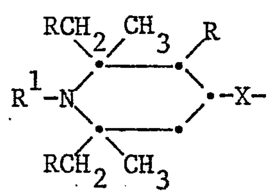

III

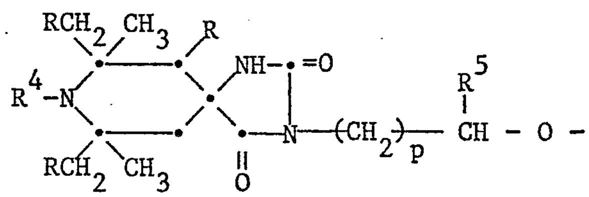

IV

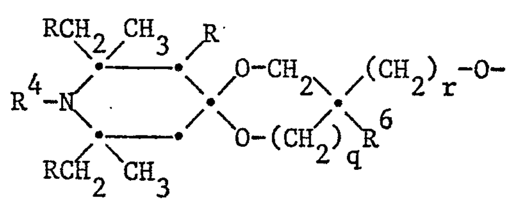

V

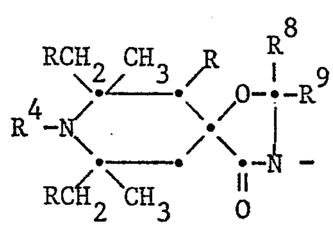

VI

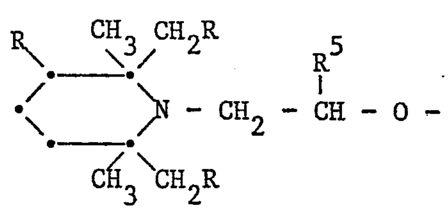

VII

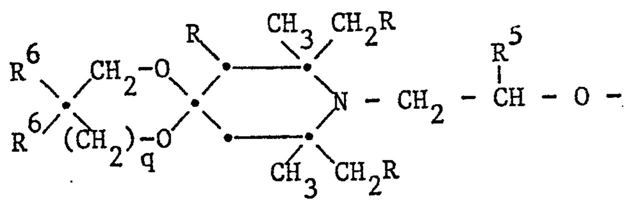

VIII

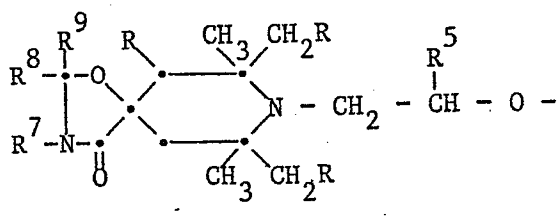

IX

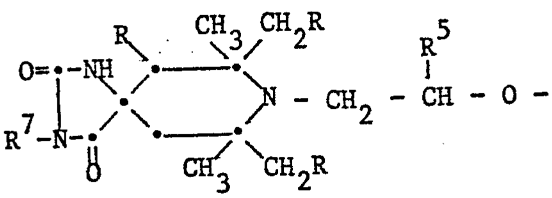

X.,

worin

R Wasserstoff oder $C_1$-$C_4$-Alkyl ist,

$R^1$ $C_1$-$C_{18}$-Alkyl, $C_3$-$C_{12}$-Alkenyl, $C_3$-$C_5$-Alkinyl, $C_7$-$C_{15}$-Aralkyl, $C_1$-$C_{18}$-Alkanoyl, $C_3$-$C_5$-Alkenoyl, Benzoyl, $C_3$-$C_{10}$-Alkoxycarbonylalkyl, Cyanomethyl oder Oxyl-Sauerstoff ist,

X -O-, -NH-, $-NR^2-$, $-OCH_2CH_2CH_2NH-$ oder $-CH_2CH_2NH-$ bedeutet, wobei $R^2$ $C_1$-$C_{18}$-Alkyl, $C_5$-$C_{12}$-Cycloalkyl oder -Alkylcycloalkyl, $C_7$-$C_{16}$-Aralkyl, $C_4$-$C_{15}$-Aryl oder Alkaryl, $C_3$-$C_{12}$-Mono-, Di- oder Trioxaalkyl,

oder eine Gruppe der Formel

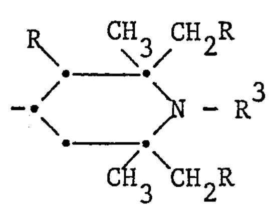

ist, wobei $R^3$ $C_1$-$C_{12}$-Alkyl, $C_3$-$C_5$-Alkenyl, $C_7$-$C_{12}$-Aralkyl, $C_1$-$C_{12}$-Alkanoyl oder Cyanomethyl darstellt,

$R^4$ $C_1$-$C_{18}$-Alkyl, $C_3$-$C_5$-Alkenyl, $C_3$-$C_5$-Alkinyl, $C_7$-$C_{12}$-Aralkyl, $C_1$-$C_{12}$-Alkanoyl, $C_3$-$C_5$-Alkenoyl, Benzoyl oder Cyanomethyl ist,

$R^5$ Wasserstoff, $C_1$-$C_4$-Alkyl, $C_1$-$C_4$-Alkoxy, Phenoxy, Tolyloxy oder

Phenyl ist,

p 1 oder 2 und q 1 oder null ist,

r im Falle von q = 1 null oder 1 ist, und im Falle von q = 0 die Zahl 1 ist,

$R^6$ H, Methyl oder Ethyl ist,

$R^7$ Wasserstoff, $C_1$-$C_{12}$-Alkyl, $C_3$-$C_5$-Alkenyl oder $C_7$-$C_{12}$-Aralkyl ist,

$R^8$ $C_2$-$C_{12}$-Alkyl, Cyclohexyl, Benzyl, Phenyl, Tolyl und

$R^9$ $C_1$-$C_{12}$-Alkyl ist, oder

$R^8$ und $R^9$ zusammen $C_4$-$C_{12}$-Alkylen sind,

D ein zweiwertiger Piperidinrest der Formel XI ist

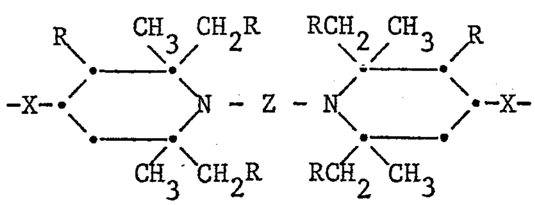 XI,

worin

Z $C_2$-$C_{12}$-Alkylen oder -Alkenylen, Xylylen oder eine Gruppe der Formel -CO-NH-B-NH-CO- oder $-CH_2-CH(R^5)-O-CO-R^{10}-CO-O-CH(R^5)-CH_2$ bedeutet, worin $R^{10}$ $C_2$-$C_{12}$-Alkylen, $C_2$-$C_4$-Alkenylen, $C_5$-$C_7$-Cycloalkylen, $C_6$-$C_{12}$-Arylen oder -NH-B-NH- ist und

B den (m + 1)-wertigen Rest eines aliphatischen, cycloaliphatischen oder aromatischen Polyisocyanates mit 4-40 C-Atomen bedeutet.

2. Verbindungen der Formeln Ia und IIa

$$A' - \overset{O}{\overset{\|}{C}} - NH - B - (NCO)_m \qquad Ia$$

$$(OCN)_m - B - NH - \overset{O}{\overset{\|}{C}} - D' - \overset{O}{\overset{\|}{C}} - NH - B - (NCO)_m \qquad IIa,$$

worin A' ein einwertiger Piperidinrest der Formel XII ist,

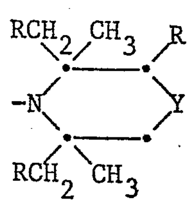 XII

und Y eine Gruppe $>C=O$, $>CH_2$, $>CH-X-\overset{O}{\overset{\|}{C}}-R^{11}$,

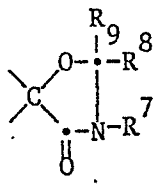 , 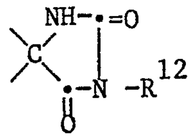 , 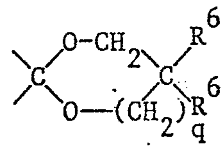

darstellt, worin $R^{11}$ $C_1$-$C_{18}$-Alkyl, $C_2$-$C_8$-Alkenyl, $C_7$-$C_{15}$-Aralkyl, $C_5$-$C_{12}$-Cycloalkyl, $C_6$-$C_{15}$-Aryl oder -Alkylaryl, -NH-$R^{13}$ oder -N($R^{13}$)$_2$ darstellt und $R^{13}$ $C_1$-$C_{12}$-Alkyl, Benzyl, Allyl, Cyclohexyl, $C_6$-$C_{15}$-Aryl oder -Alkaryl ist, $R^{12}$ Wasserstoff, $C_1$-$C_{12}$-Alkyl, $C_3$-$C_5$-Alkenyl oder $C_7$-$C_{12}$-Aralkyl ist,

D' ein zweiwertiger Piperidinrest der Formel XIII bis XV ist,

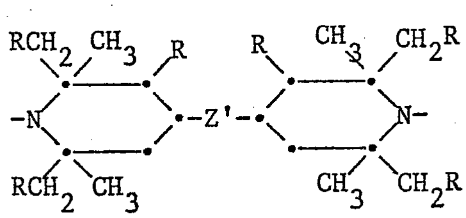

XIII

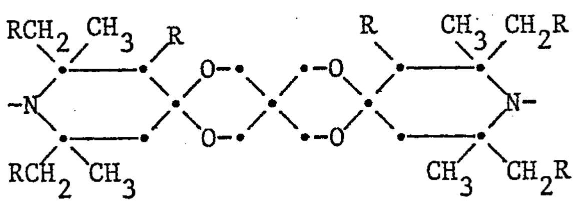

XIV

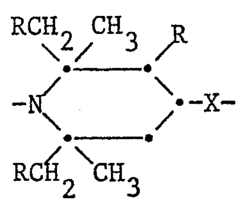

XV

worin

Z' einer der Reste -N($R^{14}$)-, -N($R^{15}$)-CO-N($R^{15}$)-, -N($R^{15}$)-CO-CO-N($R^{15}$)-, -N($R^{15}$)-CO-$R^{16}$-CO-N($R^{15}$)-, $C_4$-$C_{10}$-Alkylen, p-Xylylen,

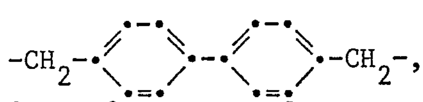

-O-$R^{17}$-O-, -O-C(=O)-$R^{16}$-C(=O)-O- oder -O-C(=O)-NH-B-NH-C(=O)-O- ist und

$R^{14}$ $C_1$-$C_{12}$-Alkyl, $C_3$-$C_5$-Alkenyl, Cyclohexyl oder $C_2$-$C_8$-Alkanoyl,

$R^{15}$ Wasserstoff, $C_1$-$C_{12}$-Alkyl, Cyclohexyl oder $C_3$-$C_8$-Alkoxyalkyl,

$R^{16}$ $C_2$-$C_{12}$-Alkylen, $C_2$-$C_4$-Alkenylen, $C_5$-$C_7$-Cycloalkylen oder $C_6$-$C_{12}$-Arylen und

$R^{17}$ $C_2$-$C_{12}$-Alkylen, 2-Butenylen-1,4 oder Xylylen bedeuten, und m, B und R die in Anspruch 1 gegebene Bedeutung haben.

3. Verbindungen gemäss Anspruch 1 oder 2 der Formeln I, II, Ia oder IIa, worin m 1 ist und B einen der zweiwertigen Reste Toluylen-2,4, Toluylen-2,6, Naphthylen-1,5, Tetramethylen, Hexamethylen, Octamethylen, Dodecamethylen, 2,2,4- oder 2,4,4-Tetramethylhexamethylen oder einen Rest der Formeln

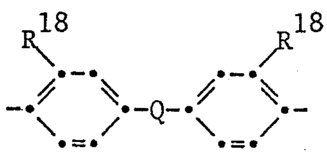 , 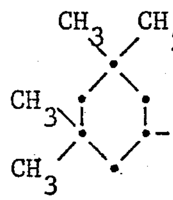 oder 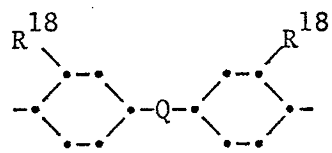

bedeutet, worin Q eine direkte Bindung, $-CH_2-$ oder $(CH_3)_2C<$ ist und $R^{18}$ H oder $CH_3$ ist.

4. Verbindungen gemäss Anspruch 3 der Formel I oder Ia.

5. Verbindungen gemäss Anspruch 3 der Formel I, worin A ein Piperidinrest der Formel III ist, in dem R Wasserstoff bedeutet.

6. Verbindungen gemäss Anspruch 4, worin $R^1$ $C_1$-$C_{12}$-Alkyl, $C_3$-$C_5$-Alkenyl, Benzyl, $C_2$-$C_4$-Alkanoyl oder Benzoyl bedeutet.

7. Verbindungen gemäss Anspruch 5, worin $R^1$ $C_1$-$C_4$-Alkyl, Allyl, Benzyl oder Acetyl bedeutet.

8. Verwendung von Verbindungen der Formel I des Anspruches 1 zum Stabilisieren von lichtempfindlichen organischen Materialien gegen Schädigung durch Lichteinwirkung.

9. Verwendung gemäss Anspruch 8 zum Stabilisieren von organischen Polymeren, insbesondere von Lackharzen.

10. Verfahren zur Herstellung von Verbindungen der Formel Ia und IIa gemäss Anspruch 2 durch Umsetzung entweder einer Verbindung der Formel A'-H mit mindestens einem Mol-Aequivalent, oder einer Verbindung der Formel H-D'-H mit mindestens zwei Mol-Aequivalenten eines Polyisocyanates der Formel $B(NCO)_{m+1}$, worin A', D' und B die in Anspruch 2 gegebene Bedeutung haben, bei niedrigen Temperaturen, vorzugsweise bei -20 bis +30°C.

11. Verfahren gemäss Anspruch 10, dadurch gekennzeichnet, dass man das Polyisocyanat im Ueberschuss verwendet.

0094343

Europäisches Patentamt

# EUROPÄISCHER RECHERCHENBERICHT

Nummer der Anmeldung

EP 83 81 0168

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,A | DE-A-2 950 067 (CHEMISCHE WERKE HÜLS AG) * Ansprüche 1-3, 5-7 * | 1,3-5, 8-11 | C 07 D 211/46<br>C 07 D 211/58<br>C 07 D 211/94<br>C 07 D 471/10<br>C 07 D 491/113<br>C 07 D 498/10<br>C 08 K 5/34<br>C 08 K 5/35 //<br>C 09 D 7/12<br>(C 07 D 471/10<br>C 07 D 235/00<br>C 07 D 221/00 )<br>(C 07 D 491/113<br>C 07 D 319/00<br>C 07 D 221/00 )<br>(C 07 D 491/113<br>C 07 D 317/00<br>C 07 D 221/00 )<br>-/- |
| | --- | | |
| A | DE-A-2 636 143 (CHIMOSA CHIMICA ORGANICA S.p.A.) * Ansprüche 1, 2, 4, 5, 12, 14 * | 2,6-8, 10,11 | |
| | --- | | |
| D,A | DE-A-2 719 132 (CIBA-GEIGY AG) * Ansprüche 1, 2, 8; Beispiele 2, 5, 6 * | 1,3,8, 10 | |
| | --- | | |
| A | DE-A-2 727 385 (CIBA-GEIGY AG) * Ansprüche 1, 10, 16 * | 1,6-8 | |
| | --- | | |
| P,A | EP-A-0 052 073 (CIBA-GEIGY AG) * Ansprüche 1-8, 11, 12 * | 1,6-9 | RECHERCHIERTE SACHGEBIETE (Int. Cl. 3) |
| | ----- | | C 07 D 211/00<br>C 07 D 471/02<br>C 07 D 471/10<br>C 07 D 491/00<br>C 07 D 498/02<br>C 07 D 498/10<br>C 08 K 5/34 |

Der vorliegende Recherchenbericht wurde für alle Patentansprüche erstellt.

| Recherchenort | Abschlußdatum der Recherche | Prüfer |
|---|---|---|
| BERLIN | 27-07-1983 | HASS C V F |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82

0094343

Europäisches Patentamt

# EUROPÄISCHER RECHERCHENBERICHT

Nummer der Anmeldung

EP 83 81 0168

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| | | | (C 07 D 498/10<br>C 07 D 263/00<br>C 07 D 221/00 ) |
| | | | RECHERCHIERTE SACHGEBIETE (Int. Cl. 3) |
| | | | |

Der vorliegende Recherchenbericht wurde für alle Patentansprüche erstellt.

| Recherchenort | Abschlußdatum der Recherche | Prüfer |
|---|---|---|
| BERLIN | 27-07-1983 | HASS C V F |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82